# EUROPEAN PATENT APPLICATION

(11) **EP 4 024 049 A1**
(43) Date of publication of application: **06.07.2022**
(21) Application number: 20903935.3
(22) Date of filing: 15.12.2020
(51) Int. Cl.: G01N 33/68, G01N 33/53

(54) **BIOMARKER COMPOSITION FOR DIAGNOSING MILD COGNITIVE IMPAIRMENT USING NASAL FLUID SAMPLE, AND METHOD FOR DIAGNOSING MILD COGNITIVE IMPAIRMENT USING SAME**

(30) Priority: 19.12.2019 KR 20190171031; 10.12.2020 KR 20200172354
(71) Applicant: Daegu Gyeongbuk Institute Of Science and Technology, Daegu 42988 (KR)
(72) Inventor: MOON, Cheil, Daegu 42060 (KR); KIM, So Yeon, Daegu 42797 (KR); SON, Go Woon, Daegu 42616 (KR); YOO, Seung Jun, Gyeongsan-si Gyeongsangbuk-do 38612 (KR); LEE, Sun Ju, Ulsan 44646 (KR)
(74) Representative: Roos, Peter
(86) International application number: PCT/KR2020/018301
(87) International publication number: WO 2021/125732

(57) **Abstract**

The present invention relates to a composition for diagnosing mild cognitive impairment for non-invasive in vitro diagnosis, and a kit including same, and more specifically, to: a biomarker composition that is for diagnosing mild cognitive impairment by using an isolated nasal fluid sample as a non-invasive biological sample and that includes the S100A9 protein or a gene encoding the same; a kit that is for diagnosing mild cognitive impairment and includes said biomarker composition; and a method for providing information required for the diagnosis of mild cognitive impairment. The biomarker composition and kit according to the present invention can diagnose mild cognitive impairment with even a small amount of nasal fluid, thus being excellent in terms of simplicity and economics, and can distinguish between Alzheimer disease patients and mild cognitive impairment patients, and thus may be used to construct a plan for treating mild cognitive impairment.

## Description

### TECHNICAL FIELD

The present invention relates to a composition for diagnosing mild cognitive impairment for non-invasive in vitro diagnosis, and a kit including the same. More specifically, the present invention relates to a biomarker composition for diagnosing mild cognitive impairment using an isolated nasal fluid sample which is a non-invasive biological sample which contains S100A9 protein or a gene encoding the same, a kit for diagnosing mild cognitive impairment including the biomarker composition, and a method for providing information required for diagnosis of mild cognitive impairment. The biomarker composition and kit according to the present invention can diagnose mild cognitive impairment with even a small amount of nasal fluid, thus being excellent in terms of simplicity and economy, and can distinguish between Alzheimer disease patients and mild cognitive impairment patients, and thus may be effectively used to construct a treatment plan for mild cognitive impairment.

### BACKGROUND ART

In the global transition to an aging society in which life expectancy is increasing and the elderly population is increasing, the incidence of senile dementia represented by Alzheimer disease, stroke, or cranial nerve diseases such as Parkinson disease is increasing significantly. According to the statistics of the Korea Institute for Health and Social Affairs, the proportion of the elderly population in Korea exceeded 7% in 2000 and Korea entered the aging society. The elderly population reached 3.97 million and the proportion of the elderly population reached 8.3% in 2003, the proportion reached 14.4% in 2019, and therefore, Korea is expected to enter a fully aged society. Degenerative brain disease is a disease that occurs in the brain as people age. The disease is known to be caused by gradual loss of function of a specific group of brain cells in the brain for reasons not well known until now and death of cranial nerve cells, which are the most important for information transmission in the cranial nervous system, problems with formation or function of synapses which transmit information between the cranial nerve cells, or abnormal increase or decrease in electrical activity of cranial nerves. The degenerative brain disease can be classified by considering main symptoms that appear and brain regions that are affected, and compared to other brain diseases, Alzheimer disease and mild cognitive impairment exhibit disease-specific protein expression, death of a specific cell group, and specific symptoms through them.

Mild cognitive impairment is an intermediate stage in a cognitive function extension line connecting normality and dementia, and the basic concept is 'a state in which cognitive decline is more severe than expected but not enough to be called dementia'. Adults over the age of 85 have a one-third chance of the risk of mild cognitive impairment, and early diagnosis is the most effective way to prevent cognitive impairment from leading to dementia.

Mild cognitive impairment and Alzheimer disease are distinguished in the following aspects. In a study using spherical harmonics (SPHARM), the difference in the shape of hippocampal cells between mild cognitive impairment and Alzheimer disease was evident. People diagnosed with mild cognitive impairment did not necessarily develop Alzheimer disease, only about 10-15% of them progressed to Alzheimer, 20% of them recovered cognitive function after 1-2 years, and 40-70% of them did not progress to dementia even after 10 years.

So far, no clear cure has been identified for Alzheimer disease, and thus most scholars are paying attention to the importance of prevention. Although patients with mild cognitive impairment are at high risk for Alzheimer disease, mild cognitive impairment is attracting attention as an important point in the prevention and treatment of Alzheimer disease in that they can restore normal cognitive function. Therefore, efforts to prevent confusion between the existing mild Alzheimer disease and mild cognitive impairment and establishment of concepts of them have been made, and these are recognized as different diseases. By being diagnosed in advance in a state of mild cognitive impairment, social and economic benefits of receiving active treatment with less economic effort are also great.

Further, mild cognitive impairment does not simply mean a pre-stage Alzheimer disease, nor does it necessarily mean a memory deficit. The types of mild cognitive impairment are broadly divided into amnestic-type mild cognitive impairment and non-amnestic type mild cognitive impairment. In more detail, it is classified into three types: a type with only memory deficits (Amnestic Type), a type with memory and other cognitive deficits (Multiple domains, mild impairment Type), and a type with non-memory deficits (Single non-memory domain type). According to the study results, the amnestic-type mild cognitive impairment is more likely to progress to Alzheimer disease, and the non-amnestic type mild cognitive impairment is more likely to progress to non-Alzheimer dementia such as Lewy body dementia or frontotemporal lobar degeneration.

Although not all mild cognitive impairments lead to Alzheimer disease, the existing methods for diagnosing mild cognitive impairment were mostly extensions of Alzheimer diagnosis, and most of them were invasive or took a lot of time and expense.

Among these methods, Short Form of Geriatric Depression Scale (GDS) or mini-mental state examination (MMSE), or specialized MRI scan is representative. In addition, in the case of a brain imaging technique using a high-resolution brain imaging device among existing diagnostic methods, brain imaging is performed on patients with suspected Alzheimer disease. Although there is an advantage of early diagnosis, this image-based diagnosis method not only requires a high cost to the patients, but also delays the detection of the disease because the diagnosis is made in a state in which brain contraction or damage has already progressed.

Another existing representative diagnostic method is to diagnose spinal fluid, which diagnoses early Alzheimer by measuring changes in the amount of beta amyloid protein in the cerebrospinal fluid. However, the cerebrospinal fluid analysis itself is not only too painful for the patients, but also has a limitation in that it is accompanied by risks.

As such, recently, while focusing on prevention rather than treatment of Alzheimer, interest has been focused on mild cognitive impairment that has the potential to progress to mild Alzheimer. In addition, interest in mild cognitive impairment is rapidly increasing in that a part of patients with mild cognitive impairment may progress to Alzheimer, but a part of them return to normal. As a result, there is a need to develop a non-invasive, convenient, and accurate method for specifically diagnosing such mild cognitive impairment.

### DISCLOSURE

### TECHNICAL PROBLEM

The inventors of the present invention have tried to develop a biomarker and a composition for diagnosis capable of diagnosing mild cognitive impairment with excellent diagnostic sensitivity through non-invasive in vitro diagnosis. The present inventors found that S100A9 protein expression was increased from a nasal fluid sample of mild cognitive impairment patients, and that in Alzheimer dementia patients, S100A9 protein expression decreased and beta amyloid oligomer protein increased. Thus, they have completed the present invention by determining that patients with mild cognitive impairment can be easily selected and diagnosed with a simple nasal fluid examination.

Accordingly, an object of the present invention is to provide a biomarker composition for diagnosing mild cognitive impairment which contains an agent for measuring an expression or activity level of S100A9 protein in an isolated nasal fluid sample, a kit for diagnosing mild cognitive impairment which includes the biomarker composition, and a method for providing information required for the diagnosis of mild cognitive impairment.

### TECHNICAL SOLUTION

In order to achieve the above object, the present invention provides a biomarker composition for diagnosing mild cognitive impairment from an isolated nasal fluid sample which is a non-invasive biological sample, the biomarker composition including S100A9 protein or a gene encoding the same.

In addition, the present invention provides a kit for diagnosing mild cognitive impairment including the composition.

In addition, the present invention provides a method for providing information on diagnosis of mild cognitive impairment, the method including (a) measuring an expression or activity level of S100A9 protein in an isolated nasal fluid sample of a patient; and (b) diagnosing the patient with mild cognitive impairment in the case that the expression or activity level of S 100A9 protein in the sample in (a) is higher than that of a sample of a normal control by comparing the expression or activity levels of the sample in (a) and the sample of the normal control.

### ADVANTAGEOUS EFFECTS

According to a composition for diagnosing mild cognitive impairment according to an aspect, it is possible to diagnose mild cognitive impairment, which is difficult to diagnose early, only with a nasal fluid sample collected using a non-invasive method. In addition, in the case of a patient who has already been diagnosed with mild cognitive impairment, the transition from mild cognitive impairment to mild Alzheimer can be tracked, so it can be effectively used to plan a treatment for mild cognitive impairment.

### BRIEF DESCRIPTIONS OF THE DRAWINGS

FIG. 1 shows results of quantitative analysis of beta amyloid oligomer, a protein expressed by all Alzheimer's patients, and the mild cognitive impairment-specific biomarker protein S100A9 from nasal fluid samples collected from a normal group, a mild cognitive impairment patient group, and an Alzheimer's patient group, respectively, as plots indicating quantification of the expression level of each protein (^{∗∗}P < 0.01, ^{∗∗∗}P < 0.001).

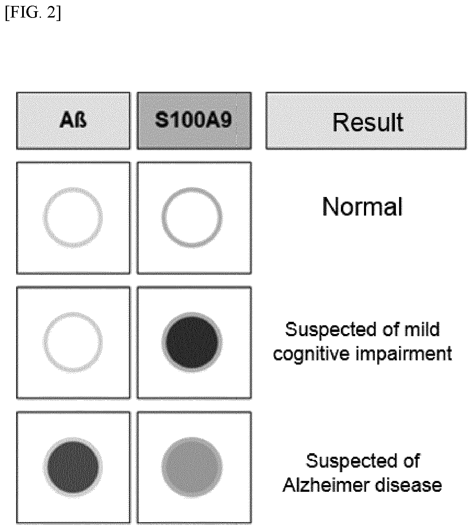

FIG. 2 is a diagram illustrating a diagnosis kit capable of diagnosing progression of mild cognitive impairment and Alzheimer from nasal fluid samples.

### BEST MODE

The present inventors have completed the present invention by finding that S 100A9 protein from an isolated nasal fluid sample can be usefully used as a composition for diagnosing mild cognitive impairment through non-invasive in vitro diagnosis. Specifically, mild cognitive impairment patients can be diagnosed and identified by measuring a concentration level of S100A9 protein, and in the case of also measuring concentration of beta amyloid oligomer protein and comparing them, it is possible to identify a patient group with Alzheimer disease and the mild cognitive impairment patients. Accordingly, the present inventors has completed the present invention by finding that, in the case of a patient who has already been diagnosed with mild cognitive impairment, treatment for mild cognitive impairment can be effectively planned by tracking the transition from mild cognitive impairment to mild Alzheimer.

Hereinafter, the present invention will be described in detail.

The present invention provides a biomarker composition for diagnosing mild cognitive impairment from an isolated nasal fluid sample as a non-invasive biological sample which includes S 100A9 protein or a gene encoding the same.

In the present invention, "Mild cognitive impairment (MCI)" is a mild condition that is not necessarily associated with the presence of dementia but is a symptom characterized by measurable impairment of thinking ability. Mild cognitive impairment frequently, but not necessarily, leads to Alzheimer disease.

"Diagnosis" of the present invention includes determining an individual's susceptibility to a specific disease or disorder, determining whether an individual currently has a specific disease or disorder, determining prognosis of an individual afflicted with a particular disease or disorder, or therametrics (for example, monitoring conditions of an individual to provide information on a treatment effect).

Further, the present invention provides a kit for diagnosing mild cognitive impairment which includes an agent for detecting S 100A9 protein or a gene encoding the same from a nasal fluid sample which is a non-invasive biological sample, as an active ingredient.

The agent may be a primer or probe that specifically binds to the S100A9 gene; or an antibody, peptide, aptamer, or compound that specifically binds to S 100A9 protein.

Further, the kit of the present invention may further include an agent for detecting beta amyloid oligomer protein or a gene encoding the same.

The agent may be a primer or probe that specifically binds to the beta amyloid oligomer gene; or an antibody, peptide, aptamer, or compound that specifically binds beta amyloid oligomer protein.

In the present invention, "primer" refers to a nucleic acid sequence having a short free hydroxyl group, capable of forming a base pair with a complementary template, and a short nucleic acid sequence functioning as a starting point for template strand copying. The primer of the present invention can be chemically synthesized using methods known in the art, such as, for example, a phosphoramidite solid support method.

In the present invention, "probe" refers to a nucleic acid fragment such as RNA or DNA consisting of several to hundreds of bases, capable of specifically binding to mRNA, and is labeled to determine whether or not a specific mRNA exists. The probe may be manufactured in the form of an oligonucleotide probe, a single-stranded DNA probe, a double-stranded DNA probe, an RNA probe, or the like, and may be labeled with biotin, FITC, rhodamine, DIG, or the like, or labeled with a radioisotope.

In the present invention, "antibody" is a term known in the art and refers to a specific immunoglobulin directed against an antigenic site. The antibody in the present invention refers to an antibody that specifically binds to S100A9 or beta amyloid oligomer of the present invention, and may be prepared according to a conventional method in the art. The form of the antibody includes polyclonal antibodies or monoclonal antibodies, and includes all immunoglobulin antibodies. The antibody refers to a complete form having two full-length light chains and two full-length heavy chains. Moreover, the antibody also includes special antibodies, such as a humanized antibody.

In the present invention, "peptide" has an advantage of high binding strength to a target material, and no denaturation occurs even during heat/chemical treatment. In addition, since the molecular size is small, it can be used as a fusion protein by attaching it to other proteins. Specifically, since it can be used by being attached to a polymer protein chain, it may be used as a diagnostic kit and a drug delivery material.

In the present invention, "aptamer" refers to a kind of polynucleotide which has a stable tertiary structure by itself, has a characteristic capable of binding to a target molecule with high affinity and specificity, and consists of a special type of single-stranded nucleic acid (DNA, RNA, or modified nucleic acid). As described above, the aptamer may specifically bind to an antigenic substance in the same way as an antibody, has higher stability than a protein, has a simple structure, and consists of a polynucleotide that is easy to synthesize, so it may be used as a substitute for the antibody.

In the present invention, "kit" may include an antibody that specifically binds to a marker component, a secondary antibody conjugate to which a label developing color by reaction with a substrate is conjugated, a chromogenic substrate solution to react with the label, a washing solution, and an enzyme reaction stop solution, and may be manufactured in a number of separate pieces of packaging or compartments containing reagent components to be used.

In the present invention, the kit may be a kit including essential elements necessary for performing RT-PCR, but is not limited thereto. For example, an RT-PCR kit may include, in addition to each pair of primers specific for a marker gene, a test tube or other suitable container, reaction buffer, deoxynucleotides (dNTPs), Taq-polymerase and reverse transcriptase, DNase, RNase inhibitors, DEPC-water, and sterile water.

The kit of the present invention may be a kit for detecting a gene for diagnosing liver cancer which includes essential elements necessary for performing a DNA chip. A DNA chip kit may include a substrate to which cDNA corresponding to a gene or fragment thereof is attached as a probe, and the substrate may include cDNA corresponding to a quantitative control gene or fragment thereof.

In addition, in the case of measuring the expression or activity levels of beta amyloid oligomer and S100A9 protein alternately from the isolated nasal fluid sample, it is possible to diagnose mild cognitive impairment more accurately. Specifically, the kit may measure the expression level of S 100A9 protein or a gene encoding the same and the expression level of beta amyloid oligomer protein or a gene encoding the same, respectively. Based on the results, in the case that only the expression level of S 100A9 protein or the gene encoding the same increases compared to a normal control, it is determined as mild cognitive impairment, and in the case that both the expression level of beta amyloid oligomer protein or the gene encoding the same and the expression level of S 100A9 protein or the gene encoding the same increase compared to the normal control, it can be determined as Alzheimer.

Accordingly, the kit may include a manual instructing to determine mild cognitive impairment in the case that only the expression level of S100A9 protein or the gene encoding the same increases compared to the normal control and to determine Alzheimer in the case that both the expression level of beta amyloid oligomer protein or the gene encoding the same and the expression level of S100A9 protein or the gene encoding the same increase compared to the normal control.

In addition, in order to increase speed and convenience of diagnosis, the kit of the present invention may be provided in an immobilized state on a suitable carrier or support using various methods known in the art. Examples of the suitable carrier or support may include agarose, cellulose, nitrocellulose, dextran, sephadex, sepharose, liposome, carboxymethyl cellulose, polyacrylamide, polyesterin, gabbro, filter paper, ion exchange resin, plastic film, plastic tube, glass, polyamine-methyl vinyl-ether-maleic acid copolymer, amino acid copolymer, ethylene-maleic acid copolymer, nylon, cups, and flat packs. Other solid substrates include cell culture plates, ELISA plates, tubes and polymeric membranes. The support may have any possible shape, for example spherical (bead), cylindrical (test tube or well inner surface), planar (sheet, test strip).

Further, the present invention provides a method for providing information on diagnosis of mild cognitive impairment, including (a) measuring an expression or activity level of S100A9 protein in an isolated nasal fluid sample; and (b) comparing the expression or activity level of S100A9 protein in the sample in (a) with that of a normal control sample and diagnosing mild cognitive impairment in the case that the expression or activity level of S100A9 protein in the sample in (a) is higher than that of the normal control sample. In the case that the measured expression or activity level of S100A9 protein is higher than that of the normal control sample, it may be determined as mild cognitive impairment.

The step of measuring the expression or activity level of S100A9 protein in the isolated nasal fluid sample may be performed according to various quantitative or qualitative immunoassay protocols developed in the prior art. Specifically, the measurement of the expression or activity level may be performed using one or more selected from the group consisting of Immunoprecipitation Assay, Complement Fixation Assay, Fluorecence Activated Cell Sorter (FACS), and a protein chip, but the present invention is not limited thereto.

Further, the step (a) may further include measuring an expression or activity level of beta amyloid oligomeris together with measuring the expression or activity level of S100A9 protein.

Furthermore, the step (b) may further include diagnosing mild cognitive impairment in the case that only the expression level of S100A9 protein or the gene encoding the same increases compared to the normal control and diagnosing Alzheimer in the case that both the expression level of beta amyloid oligomer protein or the gene encoding the same and the expression level of S100A9 protein or the gene encoding the same increase compared to the normal control to distinguish mild cognitive impairment and Alzheimer. In other words, according to the present invention, in the case of measuring the expression or activity level of beta amyloid oligomer and S 100A9 protein alternately, it is possible to diagnose mild cognitive impairment more accurately.

Therefore, according to the present invention, it is possible to diagnose and identify mild cognitive impairment patients by measuring the concentration level of S100A9 protein from an isolated nasal fluid sample, which is a non-invasive biological sample. By separately measuring the beta amyloid oligomer protein concentration and comparing it with the expression of S100A9 protein, it is possible to distinguish Alzheimer disease patients from mild cognitive impairment patients and thus it can be effectively used to plan treatment for mild cognitive impairment.

### MODES FOR CARRYING OUT INVENTION

Hereinafter, the present invention will be described in detail with reference to examples. However, the examples are for illustrative purposes only, and the scope of the present invention is not limited to these examples.

### Example 1. Selection of Subjects and Collections of Body Fluids

For a normal control, people aged 60 years or older who have normal cognitive ability on a neuropsychological test (Seoul Neuropsychological Screening Battery; SNSB) and do not meet other exclusion criteria were selected. Mild Alzheimer's patients and mild cognitive impairment patients were selected according to NINCDS/ADRDA diagnostic criteria. As the mild Alzheimer's patients, people who have deterioration in two or more cognitive domains including memory, have been identified as having problems with their daily life due to cognitive decline, not other causes, through the neuropsychological test, and do not meet other exclusion criteria were selected. The exclusion criteria are people who have been identified as having no structural problems in the brain through brain imaging test (MRI or CT), people who do not have other degenerative brain diseases, people who do not have cerebrovascular diseases that can cause cognitive impairment, people who do not have metabolic brain disease, including thyroid disease, people who do not have alcohol or drug addiction, and people who do not have head trauma or psychiatric disorders.

During the outpatient visit, the neuropsychological test and imaging test were performed at one visit for normal people and patients. After checking the test results, nasal fluid of the selected subjects was collected. The selected subjects visited a total of two times. In addition, the research was conducted with thorough information management through randomization and blindfolding so that information about normal people and patients was not exposed to those who collected samples from the subjects and those who conducted the experiment.

The subject's nasal fluid was collected by the following protocol. First, saline solution was sprayed into the nose, and then the nasal fluid was collected using a nasal inhaler and stored in a 50ml tube. In the case that the above method failed, saline was poured into the eye, and then the nasal fluid was collected and stored in a 50ml tube. The nasal fluid was stored in a -20°C refrigerator until analysis.

During the experiment, 50 µl of the sample was sonicated for 10 seconds, and target proteins were detected through Western Blot and ELISA, and diagnosis was made through quantitative comparison.

### Example 2. Diagnosis of Mild Cognitive Impairment

The S100A9 protein expression level for the diagnosis of mild cognitive impairment was analyzed in the nasal fluid sample of the patients collected in Example 1 through Western Blot.

In order to perform Western Blot, first, the frozen patients' samples were placed in cell lysis buffer containing a protease inhibitor and reacted at 4°C for 30 minutes. The reaction mixture was separated using a centrifuge at 13,000 rpm for 30 minutes, only the supernatant was extracted, and proteins extracted from the tissues were separated using 12% SDS-PAGE. The separated proteins were transferred to nitrocellulose membrane (Schleicher & Schuell, Dassel, Germany), and were blocked with blocking buffer [TBS-T (10 mM Tris-HCl, 0.15 M NaCl; pH 7.4) containing 1% skim milk] at room temperature for 1 hour. After blocking, the membrane was washed 3 times with TBS-T buffer for 10 minutes and then reacted with a primary antibody. Polyclonal rabbit Anti-S100A9 (abcam ab63818)(1:1000) as the primary antibody was mixed in TBS-T buffer containing 3% BSA and reacted at 4°C for 6 hours. As a secondary antibody, HRP-linked anti-rabbit IgG (1:5000) was reacted at room temperature for 2 hours. In each reaction, non-specific reactions were removed by washing with TBS-T three times for 15 minutes, and it was checked using ECL (Amersham).

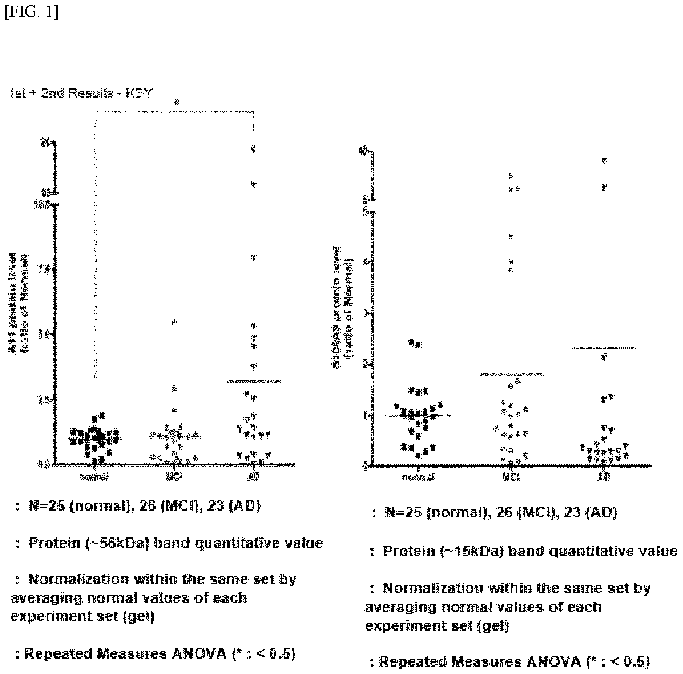

In this example, S100A9 and beta amyloid oligomer were quantitatively analyzed in the nasal fluid samples of the normal control (normal), mild cognitive impairment group (MCI), and mild Alzheimer disease group (AD), respectively. Specifically, the analysis was carried out using an antibody against S100A9 and an antibody against beta amyloid oligomer, and the results were shown in FIG. 1.

As a result, beta amyloid oligomer characteristically appeared in different lanes according to the stage of Alzheimer disease, but was expressed in all Alzheimer's patients, whereas it was not expressed in the normal and mild cognitive impairment patients. S100A9 protein was not expressed in the normal and mild Alzheimer's patients, but was expressed at a high level in mild cognitive impairment. In other words, it was found that S100A9 protein was expressed in the mild cognitive impairment patients, but did not show a significant level of expression change in the mild Alzheimer disease patients. Therefore, it was found that S100A9 protein could be used as a biomarker for diagnosing mild cognitive impairment. Further, since beta amyloid oligomer was expressed in all Alzheimer's patients regardless of the stage of Alzheimer disease, it was found that it could be utilized as a biomarker for diagnosing Alzheimer disease. Furthermore, in the case that the expression or activity levels of beta amyloid oligomer and S100A9 protein are measured alternatively, mild cognitive impairment can be diagnosed more accurately.

## Claims

1. A biomarker composition for diagnosing mild cognitive impairment using an isolated nasal fluid sample which is a non-invasive biological sample, the biomarker composition comprising S100A9 protein or a gene encoding the same.

2. A kit for diagnosing mild cognitive impairment comprising an agent capable of detecting S100A9 protein or a gene encoding the same from a nasal fluid sample which is a non-invasive biological sample, as an active ingredient.

3. The kit for diagnosing mild cognitive impairment according to claim 2, wherein the agent is a primer or probe which specifically binds to the S100A9 gene; or any one of an antibody, peptide, aptamer, or compound which specifically binds to the S100A9 protein.

4. The kit for diagnosing mild cognitive impairment according to claim 2, wherein the kit further comprises an agent capable of detecting beta amyloid oligimer protein or a gene encoding the same.

5. The kit for diagnosing mild cognitive impairment according to claim 4, wherein the agent is a primer or probe which specifically binds to the beta amyloid oligomer gene; or any one of an antibody, peptide, aptamer, or compound which specifically binds to the beta amyloid oligomeric protein.

6. The kit for diagnosing mild cognitive impairment according to claim 2, wherein the kit comprises a manual instructing, based on results of measurement of an expression level of S 100A9 protein or the gene encoding the same and an expression level of beta amyloid oligomer protein or a gene encoding the same, to determine mild cognitive impairment in the case that only the expression level of of S 100A9 protein or the gene encoding the same increases compared to a normal control and to determine Alzheimer in the case that both the expression level of beta amyloid oligomer protein or the gene encoding the same and the expression level of S100A9 protein or the gene encoding the same increase compared to the normal control.

7. A method for providing information on diagnosis of mild cognitive impairment, the method comprising:
(a) measuring an expression or activity level of S 100A9 protein in an isolated nasal fluid sample of a patient; and
(b) diagnosing the patient with mild cognitive impairment in the case that the expression or activity level of S100A9 protein in the sample in (a) is higher than that of a sample of a normal control by comparing the expression or activity levels of the sample in (a) and the sample of the normal control.

8. The method according to claim 7, wherein (a) measuring the expression or activity level of S 100A9 protein further comprises measuring an expression or activity level of beta amyloid oligomer together with the expression or activity level of S100A9 protein.

9. The method according to claim 7, wherein (b) diagnosing the patient further comprises diagnosing the patient with mild cognitive impairment in the case that only the expression level of S100A9 protein or a gene encoding the same increases compared to the normal control and diagnosing the patient with Alzheimer in the case that both the expression level of beta amyloid oligomer protein or a gene encoding the same and the expression level of S 100A9 protein or the gene encoding the same increase compared to the normal control.

10. The method according to claim 7, wherein the expression or activity level is measured by using one or more selected from the group consisting of Immunoprecipitation Assay, Complement Fixation Assay, Fluorecence Activated Cell Sorter (FACS), and a protein chip.
